# FASCICULE DE BREVET EUROPEEN

(11) **EP 2 012 580 B1**
(45) Date de publication et mention de la délivrance du brevet: **26.09.2018**
(21) Numéro de dépôt: 07731806.1
(22) Date de dépôt: 23.03.2007
(51) Int. Cl.: A01K 47/06, A01K 53/00

(54) **PROCEDE DE TRAITEMENT POUR AMELIORER LA CONSERVATION DE PRODUITS ALTERABLES ET DISPOSITIF DE MISE EN OEUVRE**
BEHANDLUNGSVERFAHREN ZUR VERBESSERUNG DER KONSERVIERUNG VON PRODUKTEN, DIE EINER VERÄNDERUNG UNTERLIEGEN, SOWIE GERÄT ZUR DURCHFÜHRUNG DIESES VERFAHRENS
TREATMENT METHOD TO IMPROVE THE PRESERVATION OF ALTERABLE PRODUCTS AND IMPLEMENTATION DEVICE

(30) Priorité: 21.04.2006 FR 0651412
(43) Date de publication de la demande: 14.01.2009
(73) Titulaire: ABEILLES SANTE, 65110 Cauterets (FR)
(72) Inventeur: BALLOT, Philippe, 65700 Lahitte-Toupiere (FR); FLURINS Catherine, 65700 Lahitte-Toupiere (FR)
(74) Mandataire: A.P.I. Conseil
(86) Numéro de dépôt international: PCT/FR2007/050993
(87) Numéro de publication internationale: WO 2007/122337

(56) Documents cités:
- EP-A1- 0 109 993
- EP-A1- 1 547 472
- FR-A1- 2 607 357
- US-A- 2 496 285
- US-A- 3 978 534

## Description

L'invention concerne un procédé de traitement pour améliorer la conservation de produits altérables dans le temps.

L'invention concerne également un dispositif utilisé pour la mise en oeuvre du procédé.

Le document US3 978 534 ne traite pas de la conservation de produits altérables dans le temps mais il décrit une solution pour augmenter le rendement de la fabrication en miel d'une ruche. Pour cela, il décrit une ruche équipée d'un distributeur de nourriture pour abeilles comportant un réservoir de produit spiritueux d'alimentation des abeilles en communication avec une rigole dans laquelle le sirop s'écoule et les abeilles viennent se nourrir. Ainsi, les abeilles se nourrissent d'un sirop qu'elles stockent dans les alvéoles des cadres de la ruche pour nourrir la colonie. Ce stockage de nourriture déclenche un stimulus pour accroitre la population. La population augmentant, le rendement s'en trouver amélioré.

L'invention s'applique à la conservation de produits et tout particulièrement à la conservation de produits à ingérer, de produits cosmétiques et de produits de santé.

L'invention s'applique à tout procédé industriel de fabrication de produits à ingérer ou de produits de santé ou de produits cosmétiques afin d'améliorer les pouvoirs de conservation de façon naturelle, c'est-à-dire sans conservateur utilisé usuellement et en particulier sans produit chimique.

L'invention s'applique également à des produits entrant dans la fabrication des produits à ingérer ou de produits de santé ou de produits cosmétiques.

La présente invention apporte une solution écologique et économique au problème de la conservation de produits altérables dans le temps.

Classiquement, ce problème est résolu par l'introduction de conservateurs chimiques dans les produits ou l'utilisation d'un procédé de pasteurisation dans le cas de certains produits liquides.

La présente invention s'écarte radicalement des solutions classiques. En effet, le procédé de traitement pour améliorer la conservation de produits comprend selon l'invention, l'exposition des produits pendant une durée prédéterminée dans un espace contenant une source d'émission de vibrations produites naturellement par une colonie d'abeilles, selon les caractéristiques du procédé de la revendication 1 et dispositif de la revendication 11.

Les produits peuvent être des produits à ingérer (produits alimentaires ou médicaments ou diététiques), des produits cosmétiques, des produits de santé (baumes, pommades, spray, pansements) ou des produits introduits dans un produit à ingérer ou d'un cosmétique ou d'un produit de santé pour en améliorer leur pouvoir de conservation.

Les produits introduits sont des matières apicoles non habituellement récoltées (couvain, venin, bois de ruche, larves d'abeilles).

Certaines matières apicoles sont inutilisables dans des applications industrielles telles que la fabrication de produits à ingérer ou de produits de santé à appliquer sur le corps ou la fabrication de cosmétiques, du fait de leur rareté pour certaines ou du fait de leur toxicité pour d'autres.

Les matières apicoles sont les matières de la ruche comme par exemple le venin d'abeille ou le bois de ruche ou le couvain ou les larves elles-mêmes ou la cire d'abeille etc.

Or, le déposant s'est aperçu également après de nombreux essais, que les matières apicoles améliorent aussi la conservation des aliments ou des produits cosmétiques dans lesquels elles sont incorporées.

Il est proposé selon une autre caractéristique du procédé de traitement d'exposer une matière apicole et de l'introduire dans un produit afin d'augmenter encore la durée de conservation du produit. Le produit peut être un produit à ingérer ou un produit cosmétique ou un produit de santé.

Le problème technique rencontré pour l'exploitation des matières apicoles a été de supprimer totalement tout risque de toxicité et de rendre l'exploitation industrielle même dans le cas de rareté de la matière sans que les coûts ne deviennent pour autant prohibitifs.

Ce problème est résolu selon une caractéristique du procédé en utilisant ces matières après dilution.

La dilution est réalisée par étapes successives jusqu'à 1/10¹⁰ (1/10 puissance10).

En effet, le procédé proposé permet l'usage de matières apicoles dans un procédé industriel de fabrication de produits à ingérer ou de produits de santé ou de produits cosmétiques afin d'améliorer les pouvoirs de conservation de façon naturelle, c'est-à-dire sans conservateur utilisé usuellement et en particulier sans produit chimique.

Selon le procédé, les matières apicoles ainsi traitées sont incorporées avec des doses homéopathiques dans les produits (donc en très faibles quantités), résolvant ainsi le problème de rareté et réduisant tout risque pour le corps humain (cas du venin d'abeille) tout en permettant une amélioration du pouvoir de conservation du produit obtenu à l'issue de ce procédé et également des produits dans lesquels elles sont incorporées.

Le traitement de matière apicole comprend les étapes suivantes,
- la récupération ou l'extraction de matière apicole dans une quantité prédéterminée,
- l'exposition de cette matière apicole pendant une durée prédéterminée dans une zone à l'intérieur d'un dispositif comprenant un espace contenant une source d'émission de vibrations produites naturellement par une colonie d'abeilles,
- le retrait de la matière apicole du dispositif et la dilution dans de l'eau avec un taux de dilution d'au moins 1/10ⁿ, n étant supérieur ou égal à 10.
- l'introduction de la dilution dans une proportion prédéterminée pour un volume donné de produit à ingérer ou de produits de santé ou de produit cosmétique.

Pour des raisons de facilité de mise en oeuvre, la matière exposée est dans un emballage.

On a obtenu la conservation d'une crème à usage cosmétique pendant 36 mois, en exposant de la matière apicole dans le dispositif pendant 24 heures et en utilisant cette matière en dilution dans la crème, l'apport de cette matière diluée étant dans une proportion de 0,2 ml de dilution dans 30 litres de crème.

Pour obtenir la conservation de produits cosmétiques, la matière apicole utilisée est dans les exemples donnés du bois de ruche ou du couvain. Il pourra s'agir de cire ou de larves d'abeilles ou autre matière apicole.

Le bois de ruche est utilisé plus particulièrement pour conserver un produit cosmétique tel qu'une crème antivieillissement de la peau (crème anti-age).

Le couvain est utilisé plus particulièrement pour conserver un produit cosmétique tel qu'une crème de soin pour peau sensible.

La matière apicole plus particulièrement utilisée dans la fabrication de produits à ingérer ou à appliquer sur le corps pour l'amélioration du pouvoir de conservation de ces produits, est le venin d'abeille.

Le procédé permet en outre la fabrication d'une dilution de venin d'abeille (élixir venin) ayant un bon pouvoir de conservation sans additif, pouvant entrer dans la fabrication d'un produit à ingérer ou à appliquer sur le corps.

Selon le procédé, les vibrations émises sont dans une gamme de 15 à 250Hz.

Pour obtenir les meilleurs résultats quant à la durée de conservation, les produits sont placés dans un rayon inférieur ou égal à 2m de la source d'émission.

L'invention concerne également un dispositif de mise en oeuvre du procédé comprenant un habitacle comportant une zone d'exposition de produits et au moins une source d'émission de vibrations produites naturellement par une colonie d'abeilles.

L'habitacle peut être formé par une ruche comportant un corps et une zone d'exposition.

La taille de l'habitacle peut être adaptée au traitement de grands volumes de produits, cet habitacle se présentera alors sous la forme d'un local comprenant une ou plusieurs zones d'exposition et une ou plusieurs ruches peuplées chacune d'une colonie d'abeilles vivantes.

D'autres particularités et avantages de l'invention apparaîtront clairement à la lecture de la description qui est faite ci-après et qui est donnée à titre d'exemple illustratif et non limitatif et en regard des figures sur lesquelles :
- La figure 1 représente un premier mode de réalisation du dispositif selon l'invention ;
- La figure 2A représente un deuxième mode de réalisation du dispositif selon l'invention ;
- la figure 2B représente une vue de dessus couvercle retiré du dispositif de la figure 2A ;
- la figure 3 représente le schéma en coupe d'une variante d'exécution du deuxième mode de réalisation ;
- La figure 4 représente un troisième mode de réalisation du dispositif selon l'invention ;
- la figure 5 représente une vue de dessus à l'intérieur du local de la figure 4 ;

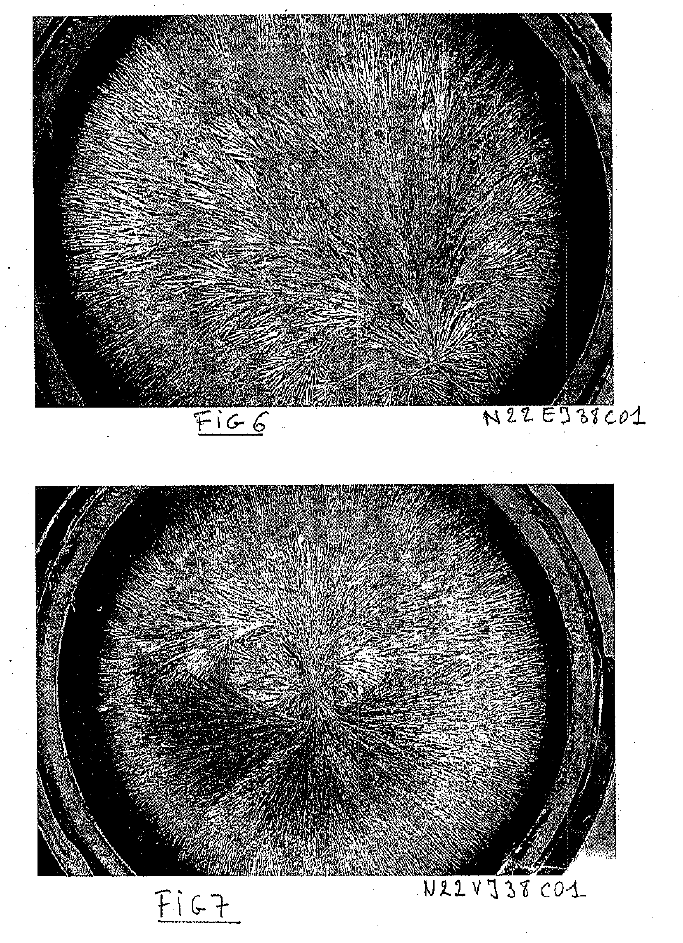

- les figures 6 et 7 illustrent respectivement une image de cristallisation sensible dans l'exemple du venin d'abeille, obtenues pour les échantillons N22EJ38CO1 et N22VJ38CO1 du rapport d'étude annexé à la description, la dilution de venin étant dénommé Elixir Venin dans ce rapport.

Le procédé de traitement proposé permet d'améliorer la conservation de produits altérables dans le temps tel que des produits comestibles alimentaires ou médicaments ou produits diététiques. Le procédé permet également d'améliorer la conservation de produits cosmétiques ou de produits de santé. Selon l'invention le traitement consiste à exposer les produits pendant une durée prédéterminée dans un espace contenant une source d'émission de vibrations produites naturellement par une colonie d'abeilles. La durée d'exposition est prédéterminée selon les volumes (ou quantité de produits) à traiter mais aussi selon la durée de conservation désirée. Dans tous les cas la durée d'exposition est d'au moins une dizaine d'heures.

Le procédé de traitement permet d'améliorer la conservation de produits tels que des matières apicoles et, en introduisant dans d'autres produits cette matière apicole traitée selon le procédé, d'en améliorer également leur conservation.

La description détaillée donnée à titre d'exemple dans la suite correspond à l'application du procédé à des matières apicoles, le procédé comprenant alors une étape supplémentaire consistant à introduire la matière apicole exposée dans un produit pour lui augmenter son pouvoir de conservation.

Le procédé de traitement comprend plus précisément les étapes suivantes :
- la récupération ou extraction de matière apicole dans une quantité prédéterminée,
- l'exposition de cette matière apicole pendant une durée prédéterminée dans une zone à l'intérieur d'un dispositif comprenant un espace contenant une source d'émission de vibrations produites naturellement par une colonie d'abeilles,
- le retrait de la matière apicole du dispositif et la dilution dans de l'eau avec un taux de dilution d'au moins 1/10ⁿ, n étant supérieur ou égal à 10.
- l'introduction de la dilution dans une proportion prédéterminée pour un volume donné de produit à ingérer ou de produits de santé ou de produit cosmétique.

Le dispositif permettant de mettre en oeuvre le procédé comporte un habitacle comprenant au moins une zone d'exposition et au moins une source d'émission de vibrations produites naturellement par une colonie d'abeilles.

Dans les deux premiers modes de réalisations l'habitacle est réalisé par une ruche qui comporte un corps de ruche et la colonie d'abeilles et une zone d'exposition.

Dans le troisième mode de réalisation l'habitacle est réalisé par un local comprenant une ou plusieurs source(s) d'émission de vibrations produites naturellement par une colonie d'abeilles et une ou plusieurs zones d'exposition. Les sources d'émission de vibrations produites naturellement par une colonie d'abeilles seront réalisées par un ou plusieurs corps de ruches peuplées d'abeilles vivantes.

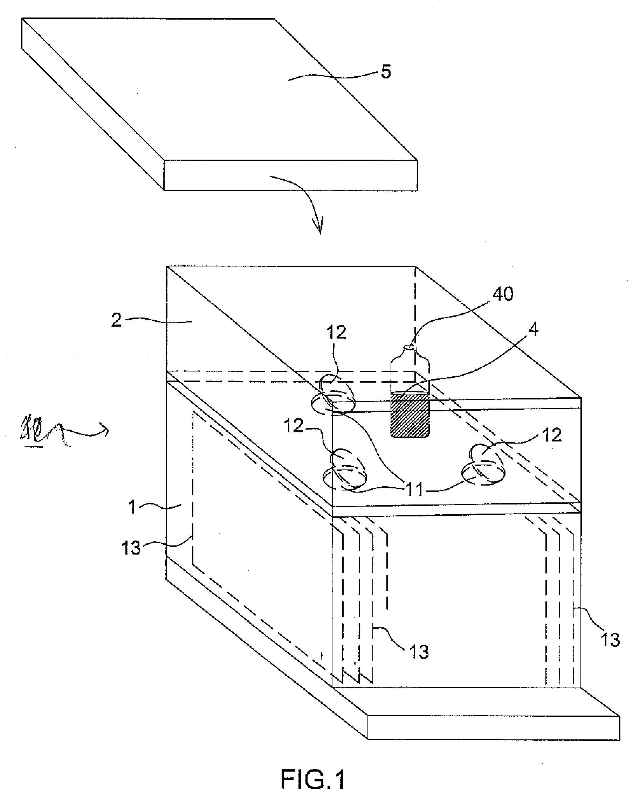

La figure 1 illustre le mode de réalisation d'un dispositif permettant plus particulièrement de mettre en oeuvre l'étape d'exposition de la matière apicole à traiter. Ce dispositif peut également être utilisé pour conserver des dilutions préparées. Mais dans ce cas on préférera utiliser le dispositif décrit dans le troisième mode de réalisation.

La matière traitée peut être dans un emballage, un récipient fermé, un flacon ou autre. L'emballage peut être en plastique ou en verre.

Dans le premier mode de réalisation, le dispositif comporte un corps de ruche peuplé 1 d'abeilles vivantes soit une colonie d'environ 40 000 abeilles, sur lequel on a adapté une hausse 2. Le corps de ruche comporte de façon classique et connue, des cadres 13 juxtaposés et au moins un accès vers l'extérieur (extérieur du dispositif).

Un couvre cadres 10 percé de passages 11 pour les abeilles est interposé entre le corps de ruche et la hausse. Les passages 11 sont refermés à l'aide de clapets 12 lorsque le dispositif n'est pas utilisé. Le ou les produit(s) apicoles 4 à traiter (traitement également dénommé dynamisation de la matière) sont placés dans un emballage 40 puis dans le hausse 2 en ouvrant et refermant le toit 5. L'emballage peut se présenter sous la forme d'un ou de contenant(s) hermétiques 40. Dans le cas où la matière est du venin d'abeille, ce venin sera dans un flacon fermé.

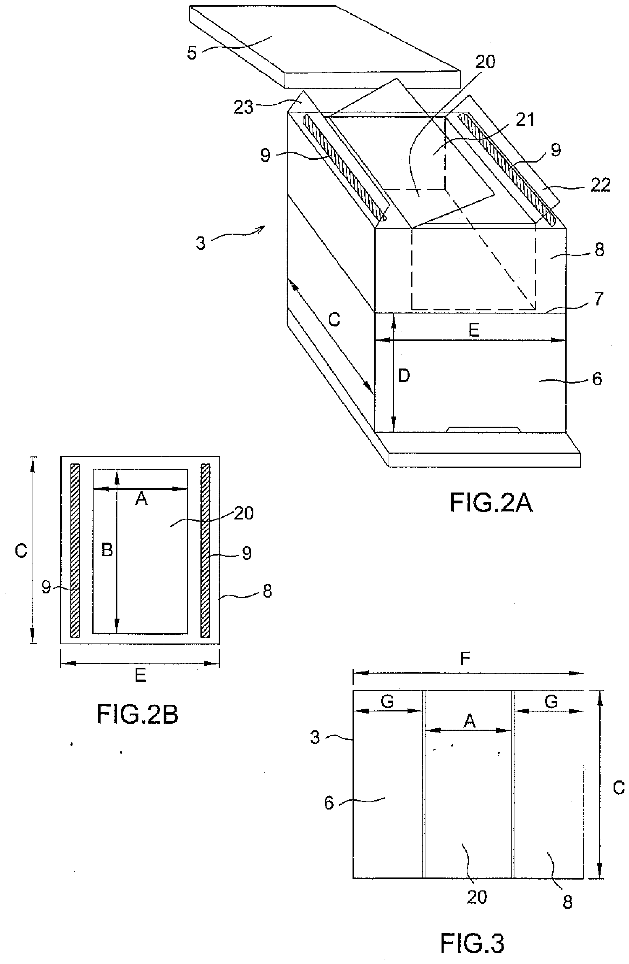

Dans le deuxième mode de réalisation schématisé par les figures 2 et 3, le dispositif se présente sous la forme d'une caisse 3 comportant un premier corps de ruche 6 avec des cadres et une colonie d'abeilles (environ 40 000 abeilles); une grille à reine 7 à la place du couvre cadres percé 10 du premier mode de réalisation; un deuxième corps de ruche 8 ou une deuxième hausse. Le dispositif comporte en outre des cadres de rive 9 de part et d'autre des produits à traiter (dynamiser) ou à conserver, placé dans un caisson amovible 20. Il comporte également le caisson 20, fermé d'un couvercle 21 indépendant de celui de la ruche, contenant les produits à dynamiser ou à conserver.

Le dispositif est muni de trois couvercles indépendants 21, 22, 23. Le couvercle 21 permet l'accès au caisson 20 sans mettre l'utilisateur en contact avec les abeilles. Le deuxième corps de ruche 8 ou hausse est fermé par le toit 5 de la ruche.

A titre d'exemple, les dimensions du dispositif peuvent être les suivantes : largeur du caisson A =280 mm, longueur du caisson B = 460 mm, longueur de la ruche C = 505 mm.

Dans la variante d'exécution illustré par la figure 3, représentant un schéma d'une coupe du dispositif, il est prévu que le caisson amovible 20 soit logé entre les deux corps de ruche 6, 8. Cette variante présente l'avantage de procurer une symétrie dans le traitement des produits ou matières placés dans le caisson.

A titre d'exemple la largeur de la ruche F = 740mm, la largeur des corps de ruche G= 225mm, La largeur du caisson A = 280mm.

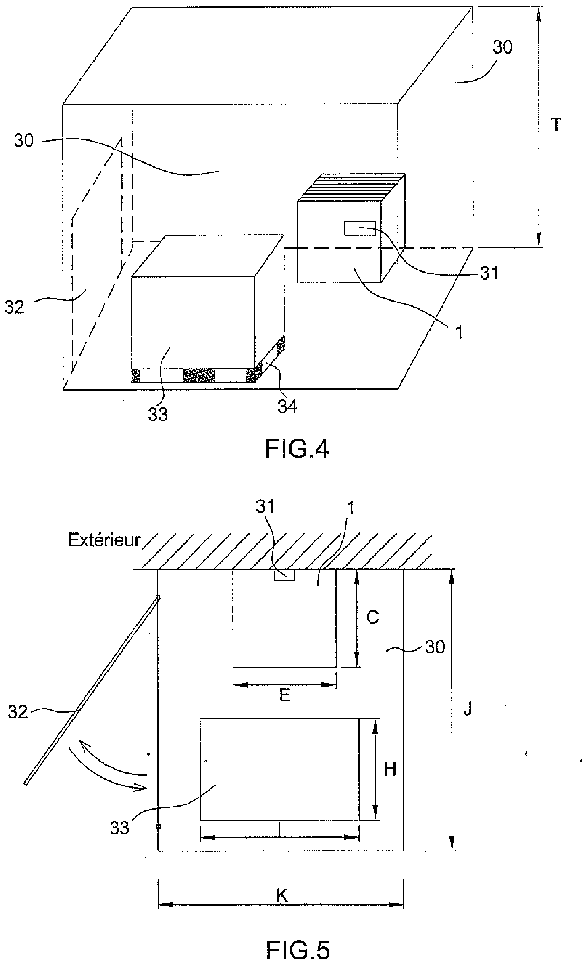

Selon le troisième mode de réalisation schématisé par les figures 4 et 5, le dispositif se présente sous la forme d'un local de stockage 30 pouvant contenir une ou plusieurs ruche(s) peuplée(s) 1.

Ce mode de réalisation est adapté au stockage de produit à traiter (à dynamiser) mais également au stockage et à la conservation de produits cosmétiques, de produits à ingérer ou de produit de santé. Ainsi, le local permet le stockage et la conservation de produits cosmétiques ou alimentaires ou de santé contenant des dilutions obtenues par le procédé de l'invention.

Les produits sont placés à côté de la ruche ou des ruches (maximum 1 à 2 mètres) et les abeilles sortent de la ruche par un accès 31 ménagé dans un mur du local, vers l'extérieur. L'espace entre la ruche et les produits stockés permet l'intervention dans la ruche si besoin. Cette variante permet de communiquer la vitalité des abeilles à un stock de produits 33 plus important ne pouvant être contenu dans la ruche elle-même. Une porte 32 permet le passage d'au moins une palette 34 avec les produits et son chariot. On prévoit une ruche par palette de produits.

A titre d'exemple les dimensions du dispositif peuvent être les suivantes, longueur de la ruche C = 505mm, largeur de la ruche E =410 mm, longueur de la zone de stockage H =800mm, largeur de la zone de stockage I=800mm, longueur du local J= 2000mm, largeur du local K= 2000mm, hauteur du local T= 2000mm.

Le procédé selon l'invention est illustré à partir des exemples suivants :

### Exemple 1: Conservation des produits cosmétiques.

- conservation de la Crème I anti-vieillissement commercialisée par le déposant sous le nom commercial de « crème de l'Apicultrice ® » Anti-Age .
- Crème II pour peaux sensibles commercialisées par le déposant sous le nom commercial de « crème de l'Apicultrice ® » Peau Sensible

Du bois de ruche a été utilisé pour la crème I et du couvain pour la crème II.
- On introduit chaque matière apicole dans le dispositif pendant 24 heures puis on a dilué à 1/10¹⁸.
- On ajoute 0,2 ml de la dilution dans 30 litres de crème.

On obtient alors une excellente conservation des crèmes, pendant 36 mois, en l'absence de tout conservateur et de tout produit chimique.

### Exemple 2 : le venin d'abeille.

Le venin d'abeille a une toxicité en application cutanée et n'est pas autorisé dans des produits à ingérer.

Cependant il est reconnu par la communauté scientifique pour ses actions positives sur :
Le coeur : il régule le rythme cardiaque et tonifie l'énergie du coeur ; il facilite la circulation du sang
Le système nerveux : il diminue la douleur locale.
Sur les glandes cortico-surrénales : il stimule leur activité.
Sur le système immunitaire : il élève le taux d'anticorps.

Le procédé proposé permet de résoudre le problème de sa toxicité en le rendant disponible à tous sous forme cutanée ou à ingérer et de résoudre le problème de sa conservation pour la fabrication de produits de santé, alimentaires ou cosmétiques. Les produits de santé peuvent se présenter sous forme de pommades ou baume à appliquer sur le corps ou sous forme de produits à ingérer.

On récupère le venin de manière à introduire le contenu de 6 dards soit 0,6 mg de venin dans 370 ml d'eau. Le mélange est ensuite mis 24 heures dans le dispositif (fig. 1) puis dilué à 1/10¹⁸.

Les résultats procurés par le procédé ont été soumis à des contrôles par un laboratoire d'analyse de cristallisation sensible. Les résultats sont visibles sur les tests de cristallisation sensible : les caractéristiques du venin sont amplifiées et la conservation de la dilution aqueuse de venin est excellente. Le rapport d'étude faite par ce laboratoire est annexé à la description. La dilution de venin se conserve 12 mois sans aucun additif.

Le procédé et le dispositif décrits permettent de transformer qualitativement un produit (alimentaire ou cosmétique ou de santé) en induisant une meilleure conservation, un meilleur effet sur la santé ou sur la vitalité ou un meilleur goût, autrement dit le dynamiser.

L'amélioration de la qualité d'un produit alimentaire ou cosmétique ou de la santé fait généralement appel à des techniques d'analyse et de maîtrise des risques microbiologiques et physico-chimiques.

Le dispositif selon l'invention permet de compléter les techniques actuelles. Cette qualité vitale peut être vérifiée par comparaison d'images de cristallisation et d'autres méthodes d'approche du monde du vivant.

Un des avantages du procédé est qu'il ne fait pas courir de risque microbiologique et physico-chimique au produit. Un autre avantage est qu'il préserve la vie des abeilles.

Les dispositifs proposés permettent d'accueillir des produits pendant une durée de quelques heures, quelques jours à plusieurs semaines.

### ANNEXE.

### Rapport d'étude de cristallisation sensible effectué sur le venin d'abeille : Elixir Venin

### I. RESUME

*Les images de Cristallisations Sensibles (I.C.S.) permettent de visualiser, indirectement, des différences énergétiques entre une haute dilution d'extrait de ruche, composant l'Elixir Venin et l'eau témoin de cette préparation.*

### II. OBJET DE LA RECHERCHE

Nous avons reçu par la poste un colis contenant, entre autres, deux flacons identiques contenant : « l'Elixir Venin », lot 051206 et l'eau pure témoin (prélèvement du 31 01 06) utilisée par l'apiculteur pour la préparation de cet Elixir Venin.

### III. MÉTHODOLOGIE

Mode opératoire habituel pour ce genre de comparaison : nous ne pouvons pas tester directement les liquides ne possédant pas de protéine.

Nous utilisons des substances organiques comme supports de visualisation des comparaisons. Ces substances sont mises en présences des eaux que l'on veut comparer et les extraits aqueux organiques, à comparer, sont conservés ensemble dans des flacons identiques.

En fait nous étudions comment des eaux diverses influencent la qualité énergétique de la substance choisie comme support de visualisation.

De la nature et de l'intensité de cet impact de l'eau sur la substance nous en déduisons quelques informations qualitatives ou énergétiques.

Nous choisissons 3 substances organiques, que nous connaissons bien, et si les résultats sont analogues ou cohérents pour les 3 expérimentations nous nous en tenons là et établissons un compte- rendu dans lequel est formulé un jugement en rapport avec la demande.

Méthodologie habituelle décrite dans des documents de l'association PRESENCES et dans « Cristaux Sensibles » Ed. du FRAYSSE (Monclar de Quercy)

Dans la nomenclature accompagnant les images de cristallisation sensible (I.C.S) les lettres E et V désignent respectivement l'eau témoin du 31 01 06 et la dilution appelée élixir de venin.

Le chiffre suivant la lettre J correspond au vieillissement de l'extrait aqueux de la substance support, en cours d'étude, et celui, après la lettre C, à la quantité de substance ajoutée au chlorure de cuivre pour obtenir l'I. C. S.

Les ICS se comparent à concentration (quantité de substance) et vieillissement de l'extrait organique identiques.

### IV. ICONOGRAPHIE

Nombre d' étuvées réalisées pour cette étude : 9
Nombre de photos conservées : 94 états photographiques.
Iconographie sélectionnée sur CD Rom.
Possibilité d'envoi d'images couleur (papier ou bristol)

### V. RÉSULTATS ET DISCUSSION

Première expérimentation N22 : Substance support de test : miel de châtaignier d'Espagne.

Le miel a été partagé en 3 extraits à 10% de miel et 90% d'eau, conservés et étudiés dans les mêmes conditions :
Extrait témoin n°1 : le miel est dissous dans 10% d'eau Ballot-Flurin prélevée le 31 01 06 et 80% d'eau PPI Lavoisier (Eau pour préparations injectables) que nous utilisons habituellement.
Extrait témoin n°2 : le miel est dissout dans 90% d'eau PPI.
Extrait, objet de la recherche : le miel est dissout dans 10% d'Elixir Venin et 80% d'eau PPI.

Les faibles quantités de liquides envoyées nous ont fait choisir la dilution à 10% des liquides à étudier. (Par expérience nous savions que la dilution à 10% est efficace)

La substance, selon qu'elle est mise en présence de l'eau témoin ou de l'Elixir Venin, se comporte différemment. Les extraits aqueux du même miel n'ont pas la même évolution.

Au cours du vieillissement des extraits étudiés, celui avec l'Elixir Venin, par rapport aux 2 extraits témoins, donnent des ICS qui présentent une nette amplification des vacuoles des centres germinatifs, une intensification de la projection (images plus rayonnantes) et une contraction des courants de cristallisation.

On observe une maturation plus nette de l'extrait avec l'Elixir dont les ICS présentent des textures très améliorées après vieillissement.

L'amélioration des textures des ICS des extraits témoins s'observent également mais en moins prononcé.

L'amplification des vacuoles des centres germinatifs s'observent dans les végétaux plus évolués et dans ceux ayant bénéficié de processus de lumière plus intenses.

Ce type de modification indique une stimulation des forces d'organisation de type lumière dans le miel support d'étude, sous l'effet de l'Elixir Venin.

L'accentuation du phénomène de maturation de l'extrait sous l'effet de L'Elixir Venin, exprime une stimulation des forces de chaleur du miel, support d'étude.

(Dans les ICS de la substance organique, les qualités de texture sont plus en rapport avec les processus végétatifs ou les forces de croissances, issues du cosmos proche (par exemple terre et lune), tandis que les qualités de structuration des ICS (agencement des 3 zones, forme et ampleur du centre germinatif, relief, rayonnement) nous renseignent plutôt sur l'action des forces d'organisation cosmiques plus lointaines (par exemple lumière et chaleur du soleil) ou sur le niveau d'évolution de la substance végétale qui peut tendre vers le minéral ou vers l'animal. En sachant que texture et organisation sont toujours mêlées et inter dépendantes de même qu'il n'existe pas de substance dans laquelle seul un type de force se manifesterait.

L'équilibre entre ces forces antagonistes (organisation et croissance) joue un rôle déterminant dans notre appréciation de la qualité globale d'une substance.)

Deuxième expérimentation N23 et troisième expérimentation N29 :Substances supports : 2 pains bio au levain.

Extraits de pain à 10% en solution dans 90% d'eau PPI pour le 2^{ième} témoin, dans 80% d'eau PPI et 10% d'eau Ballot-Flurin pour le 1^{ier} témoin et dans 80% d'eau PPI et 10% d'Elixir Venin

Les résultats de ces 2 expérimentations sont analogues. L'Elixir Venin par rapport au témoins (eaux du 31 01 06 et eau PPI) provoque dans les 2 expériences N23 et N29, plus ou moins prononcé selon les pains, des modifications de la texture des ICS et une modification de l'évolution des extraits : les extraits avec l'Elixir Venin présentent des textures dilatées (ouverture des angles de ramification) et résistent mieux au vieillissement organique.

Les extraits de pain témoins se décomposent plus vite.

Ce type de modification du comportement de la substance, en particulier le ralentissement de la décomposition par rapport au témoin et la phase de maturation évoque l'action de processus de chaleur.

Les forces d'organisation de type chaleur du pain semblent stimulées sous l'effet de l'Elixir Venin.

Le point commun entre les résultats d'une part de l'expérimentation N22 et d'autre part ceux des expérimentations N 23 et N 29, résultats analogues entre eux, c'est la manifestation d'une stimulation des forces d'organisation de la substance ou d'une valorisation de l'expression de ces forces d'organisation de la substance.

Les forces de type chaleur pour les pains et de type lumière et chaleur pour le miel sont mises en valeur dans les extraits organiques où l'Elixir Venin à été ajouté à l'eau de la solution, dans la proportion de 10% de l'extrait.

Nous estimons ces résultats cohérents et nous en déduisons un réel apport énergétique de la haute dilution d'extrait de ruche.

Malgré l'absence d'apport matériel, l'eau de l'Elixir Venin, restée chimiquement de l'eau pure sans protéine, est qualitativement transformée par la haute dilution d'extrait de ruche dont l'action se visualise sur les substances organiques choisies, arbitrairement, comme support d'étude.

### Illustrations photos imprimées :

### Miel

N22 E J38 C01 : Témoin n°1 (Témoin essentiel) avec 10% d'eau Ballot-Flurin du 31 01 06 Centre germinatif atrophié sans vacuole et décentré. Texture dilatée et brouillée par endroit.
N22 V J38 C01 : Elixir Venin 10% .

Centre germinatif, remonté plus au centre, présentant d'amples vacuole. Structure d'ensemble plus rayonnante. Texture plus serrée et améliorée par rapport aux ICS du début d'étude.

### Pain n°1

N 23 E J17 C032 : Témoin n°1. Texture encore caractéristique de pain malgré le «feutrage » central de la texture
N 23 V J17 C032 : Elixir Venin . Texture très modifiée par une dilatation des courants de cristallisation.
N23 E J27 C04 : Témoin n°1. Les taches et dépôts signalent le début de la décomposition de l'extrait de pain.
N 23 V J27 C04 : Elixir Venin. Texture encore très dilatée mais en train de se réorganiser. Pas de trace de décomposition. Texture améliorée
N 23EJ30C048 : Témoin n°1. Les signes de décomposition s'accentuent.
N23VJ30C048 : Elixir Venin. La réorganisation s'est poursuivie parallèlement à la décomposition de l'extrait témoin. Texture très améliorée et traduisant une très bonne conservation de l'extrait organique.

### Pain n°2

N29E J10C02b :Témoin n°1.Texture plate, un peu « usée » traduisant un vieillissement de l'extrait s'exprimant par une tendance minéralisante.
**N29VJ10C02b** : **Elixir Venin. Texture plus riche, plus « végétale »**, **plus caractéristique de ce pain et traduisant une meilleur conservation de l'extrait aqueux de pain par rapport à son témoin.**

L'ensemble des études faites à ce jour par le déposant montrent que les dilutions obtenues de produits apicoles exposés ont une très grande stabilité dans le temps alors même que ces dilutions sont réalisées avec de l'eau et que l'eau est un produit qui s'altère rapidement dans le temps en l'absence de traitement particulier. En outre ces résultats montrent que les produits contenant de la matière apicole exposée et diluée ont un pouvoir de conservation sans qu'il soit nécessaire de faire appel à des moyens de conservation traditionnels.

## Revendications

1. Procédé de traitement pour améliorer la conservation de produits **caractérisé en ce qu'**il comprend l'exposition des produits pendant une durée prédéterminée dans un espace contenant une source d'émission de vibrations produites naturellement par une colonie d'abeilles ; lesdits produits à traiter étant disposés dans ledit espace de telle sorte qu'ils ne sont pas au contact de la colonie d'abeilles ; lesdits produits étant des produits à ingérer (produits alimentaires ou médicaments ou diététiques) ou des produits cosmétiques ou des produits de santé (pansements, baumes, pommades, sprays) ou des produits introduits dans un autre produit tel que des produits à ingérer (produits alimentaires ou médicaments ou diététiques) ou des produits cosmétiques ou des produits de santé (pansements, baumes, pommades, sprays) pour en améliorer leur pouvoir de conservation.

2. Procédé de traitement selon la revendication 1, **caractérisé en ce que** les produits introduits dans un autre produit pour en améliorer leur pouvoir de conservation, sont des matières apicoles non habituellement récoltée (couvain, venin, bois de ruche, larves d'abeilles).

3. Procédé de traitement selon la revendication 2, **caractérisé en ce que** les matières apicoles sont diluées avant introduction dans un autre produit.

4. Procédé de traitement selon la revendication 2, **caractérisé en ce qu'**il comprend les étapes suivantes,
- la récupération ou l'extraction de matière apicole dans une quantité prédéterminée,
- l'exposition de cette matière apicole pendant une durée prédéterminée dans une zone à l'intérieur d'un dispositif comprenant un espace contenant une source d'émission de vibrations produites naturellement par une colonie d'abeilles,
- le retrait de la matière apicole du dispositif et la dilution dans de l'eau avec un taux de dilution d'au moins 1/10ⁿ, n étant supérieur ou égal à 10,
- l'introduction de la dilution dans une proportion prédéterminée pour un volume donné de produit pour en améliorer son pouvoir de conservation.

5. Procédé de traitement selon la revendication 2, **caractérisé en ce que** pour obtenir la conservation de produits cosmétiques, la matière apicole utilisée est du bois de ruche ou du couvain.

6. Procédé de traitement selon la revendication 2, **caractérisé en ce que** pour la préparation d'une crème à usage cosmétique, l'exposition de la matière apicole dans le dispositif, utilisée en dilution dans la crème, est de 24 heures et l'apport de cette matière diluée est dans une proportion de 0,2 ml de dilution dans 30 litres de crème.

7. Procédé de traitement selon la revendication 5, **caractérisé en ce que** le bois de ruche est utilisé plus particulièrement pour conserver un produit cosmétique tel qu'une crème anti-vieillissement de la peau (crème anti-age) et le couvain est utilisé plus particulièrement pour conserver un produit cosmétique tel qu'une crème de soin pour peau sensible.

8. Procédé de traitement selon la revendication 2 et 3, **caractérisé en ce que** la matière apicole plus particulièrement utilisée sous forme diluée pour la fabrication de produits cosmétiques ou de produits à ingérer ou de produits de santé afin de leur conférer un pouvoir de conservation, est le venin d'abeille.

9. Procédé de traitement selon l'une quelconque des revendications précédentes, **caractérisé en ce que** les vibrations émises sont dans une gamme de 15 à 250Hz.

10. Procédé de traitement selon l'une quelconque des revendications précédentes, **caractérisé en ce que** les produits sont placés dans un rayon inférieur ou égal à 2m de la source d'émission.

11. Dispositif pour la mise en oeuvre du procédé selon la revendication 1, **caractérisé en ce qu'**il comprend un habitacle (1, 2) comportant une source (1) d'émission de vibrations produites naturellement par une colonie d'abeilles et une zone d'exposition (2) dans laquelle sont disposés des produits à traiter de sorte qu'ils ne sont pas au contact de la colonie d'abeilles.

12. Dispositif selon la revendication 11, **caractérisé en ce que** l'habitacle comprend un corps de ruche contenant la colonie d'abeilles et la zone d'exposition au dessus du corps de ruche.

13. Dispositif selon la revendication 11, **caractérisé en ce que** l'habitacle comprend une caisse (3) comprenant un ou plusieurs corps de ruche (6, 8) avec des cadres et un caisson amovible (20) formant la zone d'exposition.

14. Dispositif selon la revendication 11, **caractérisé en ce que** l'habitacle se présente sous la forme d'un local (30) comprenant une ou plusieurs zone(s) d'exposition (33) et une ou plusieurs source(s) d'émission de vibrations produites naturellement par une colonie d'abeilles et **en ce que** la ou les sources d'émission sont constituées par une ou plusieurs ruches (1) munie d'accès (31) vers l'extérieur du local.

## Patentansprüche

1. Behandlungsverfahren zur Verbesserung der Produktkonservierung, **dadurch gekennzeichnet, dass** dabei Produkte für eine vorbestimmte Zeit in einem Raum ausgelegt werden, der eine Emissionsquelle von Schwingungen enthält, die auf natürliche Weise von einem Bienenvolk erzeugt werden; wobei die zu behandelnden Produkte in diesem Raum so angeordnet sind, dass sie nicht mit dem Bienenvolk in Kontakt stehen; wobei es sich bei den genannten Erzeugnissen um zu verzehrende Produkte (Lebensmittel oder Arzneimittel oder diätetische Produkte oder kosmetische Produkte oder Gesundheitsprodukte (Verbände, Balsame, Salben, Sprays) oder um Produkte handelt, die in ein anderes Erzeugnis eingebracht werden, wie zu verzehrende Erzeugnisse (Lebensmittel oder Arzneimittel oder diätetische Produkte) oder kosmetische Produkte oder Gesundheitsprodukte (Verbände, Balsame, Salben, Sprays), um ihre Konservierungsfähigkeit zu verbessern.

2. Behandlungsverfahren nach Anspruch 1, **dadurch gekennzeichnet, dass** die Produkte, die zur Verbesserung der Konservierungsfähigkeit in ein anderes Produkt eingebracht werden, Imkereimaterial sind, das normalerweise nicht geerntet wird (Brut, Gift, Bienenstockholz, Bienenlarven).

3. Behandlungsverfahren nach Anspruch 2, **dadurch gekennzeichnet, dass** das Imkereimaterial vor dem Einbringen in ein anderes Produkt verdünnt wird.

4. Behandlungsverfahren nach Anspruch 2, **dadurch gekennzeichnet, dass** es die folgenden Schritte umfasst,
- Rückgewinnung oder Extraktion von Imkereimaterial in einer vorbestimmten Menge,
- Aussetzen dieses Imkereimaterials für einen vorbestimmten Zeitraum in einem Bereich im Innern einer Vorrichtung, die einen Raum mit einer Emissionsquelle von Schwingungen enthält, die auf natürliche Weise von einem Bienenvolk erzeugt werden,
- Entfernung des Imkereimaterials aus der Vorrichtung und Verdünnung in Wasser in einem Verdünnungsverhältnis von zumindest 1/10ⁿ, wobei n größer oder gleich 10 ist,
- die Einführung der Verdünnung in einem vorbestimmten Verhältnis für ein gegebenes Produktvolumen, um dessen Konservierungsfähigkeit zu verbessern.

5. Behandlungsverfahren nach Anspruch 2, **dadurch gekennzeichnet, dass** zur Konservierung von kosmetischen Produkten Imkereimaterial aus Bienenstockholz oder Brut verwendet wird.

6. Behandlungsverfahren nach Anspruch 2, **dadurch gekennzeichnet, dass** für die Herstellung einer Creme zur Verwendung in der Kosmetik das verdünnt in die Creme eingebrachte Imkereimaterial 24 Stunden ausgesetzt wird und die Einbringung dieses verdünnten Stoffes in einem Verhältnis von 0,2 ml Verdünnung in 30 Liter Creme erfolgt.

7. Behandlungsverfahren nach Anspruch 5, **dadurch gekennzeichnet, dass** das Bienenstockholz insbesondere zur Konservierung eines kosmetischen Produkts wie einer Creme gegen die Hautalterung (Anti-Ageing-Creme) und die Brut insbesondere zur Konservierung eines kosmetischen Produkts wie einer Pflegecreme für empfindliche Haut verwendet wird.

8. Behandlungsverfahren nach den Ansprüchen 2 und 3, **dadurch gekennzeichnet, dass** das Imkereimaterial, das insbesondere in verdünnter Form zur Herstellung von kosmetischen oder zu verzehrenden Produkten oder Gesundheitsprodukten verwendet wird, um ihnen eine Konservierungsfähigkeit zu verleihen, Bienengift ist.

9. Behandlungsverfahren nach einem beliebigen der vorstehenden Ansprüche, **dadurch gekennzeichnet, dass** die emittierten Schwingungen in einem Bereich von 15 bis 250 Hz liegen.

10. Behandlungsverfahren nach einem beliebigen der vorstehenden Ansprüche, **dadurch gekennzeichnet, dass** die Produkte in einem Radius von weniger als oder gleich 2 m von der Emissionsquelle platziert werden.

11. Vorrichtung zur Durchführung des Verfahrens nach Anspruch 1, **dadurch gekennzeichnet, dass** sie ein Gehäuse (1, 2) mit einer Emissionsquelle (1) von Schwingungen umfasst, die von einem Bienenvolk auf natürliche Weise erzeugt werden, und mit einer Expositionszone (2), in der die zu behandelnden Produkte so angeordnet sind, dass sie nicht mit dem Bienenvolk in Kontakt kommen.

12. Vorrichtung nach Anspruch 11, **dadurch gekennzeichnet, dass** das Gehäuse einen Bienenstockkörper umfasst, der das Bienenvolk und die Expositionszone über dem Bienenstockkörper enthält.

13. Vorrichtung nach Anspruch 11, **dadurch gekennzeichnet, dass** das Gehäuse einen Kasten (3) umfasst, der einen oder mehrere Bienenstockkörper (6, 8) mit Rahmen und einen abnehmbaren Kasten (20) umfasst, der die Expositionszone bildet.

14. Vorrichtung nach Anspruch 11, **dadurch gekennzeichnet, dass** das Gehäuse die Form eines Raumes (30) aufweist, der eine oder mehrere Expositionszone(n) (33) und eine oder mehrere Emissionsquelle(n) aufweist, die von einem Bienenvolk auf natürliche Weise erzeugt werden, und dass die Emissionsquelle(n) aus einem oder mehreren Bienenstöcken (1) besteht/bestehen, die einen Ausgang (31) nach außerhalb des Raums haben.

## Claims

1. A treatment method to improve the preservation of products **characterised in that** it comprises exposing the products for a predetermined time in a space containing an emission source of vibrations naturally produced by a bee colony; said products to be treated being arranged in said space in such a way that they are not in contact with the bee colony; said products being products to be ingested (food products or medicinal products or dietetic products) or cosmetic products or health products (bandages, balms, ointments, sprays) or products introduced into another product such as products to be ingested (food products or medicinal products or dietetic products) or cosmetic products or health products (bandages, balms, ointments, sprays) to improve the preservability thereof.

2. The treatment method of claim 1, **characterised in that** the products introduced into another product to improve the preservability thereof are beekeeping materials not usually harvested (brood, venom, hive wood, bee larvae).

3. The treatment method of claim 2, **characterised in that** the beekeeping materials are diluted before introduction into another product.

4. The treatment method of claim 2, **characterised in that** it comprises the following steps,
- recovering or extracting beekeeping material in a predetermined amount,
- exposing this beekeeping material for a predetermined time in a zone inside a device comprising a space containing an emission source of vibrations naturally produced by a bee colony,
- removing the beekeeping material from the device and diluting in water with a dilution ratio of at least 1/10ⁿ, n being greater than or equal to 10,
- introducing the dilution in a predetermined proportion for a given volume of product to improve the preservability thereof.

5. The treatment method of claim 2, **characterised in that** to obtain the preservation of cosmetic products, the beekeeping material used is hive wood or brood.

6. The treatment method of claim 2, **characterised in that** for the preparation of a cream for cosmetic use, the exposure of the beekeeping material in the device, used in dilution in the cream, is 24 hours and this diluted material is added in a proportion of 0.2 ml of dilution in 30 litres of cream.

7. The treatment method of claim 5, **characterised in that** hive wood is used more particularly to preserve a cosmetic product such as an anti-skin-ageing cream (anti-age cream) and brood is used more particularly to preserve a cosmetic product such as a beauty cream for sensitive skin.

8. The treatment method of claim 2 and 3, **characterised in that** the beekeeping material more particularly used in diluted form for the manufacture of cosmetic products or products to be ingested or health products, in order to make said products preservable, is bee venom.

9. The treatment method of any one of the preceding claims, **characterised in that** the vibrations emitted are in a range from 15 to 250 Hz.

10. The treatment method of any one of the preceding claims, **characterised in that** the products are placed in a radius less than or equal to 2 m from the emission source.

11. A device for implementing the method of claim 1, **characterised in that** it comprises an enclosure (1, 2) comprising an emission source (1) of vibrations naturally produced by a bee colony and an exposure zone (2) in which products to be treated are arranged in such a way that they are not in contact with the bee colony.

12. The device of claim 11, **characterised in that** the enclosure comprises a hive body containing the bee colony and the exposure zone above the hive body.

13. The device of claim 11, **characterised in that** the enclosure comprises a case (3) comprising one or more hive bodies (6, 8) with frames and a removable box (20) forming the exposure zone.

14. The device of claim 11, **characterised in that** the enclosure is in the form of a premises (30) comprising one or more exposure zones (33) and one or more emission sources of vibrations naturally produced by a bee colony and **in that** emission sources consist of one or more hives (1) provided with access (31) towards the outside of the premises.
